# EUROPEAN PATENT APPLICATION

(11) **EP 2 085 119 A1**
(43) Date of publication of application: **05.08.2009**
(21) Application number: 07710936.1
(22) Date of filing: 13.02.2007
(51) Int. Cl.: A61N 7/02

(54) **AN ULTRASONIC THERAPEUTIC DEVICE CAPABLE OF MULTIPOINT TRANSMITTING**

(30) Priority: 28.11.2006 CN 200610160810
(71) Applicant: Chongqing Ronghai Medical Ultrasound Industry Ltd., Yubei District Chongqing 401121 (CN)
(72) Inventor: ZHANG, Chunyan, Chongqing 401121 (CN); CHEN, Wenzhi, Chongqing 401121 (CN); YAN, Siyuan, Chongqing 401121 (CN)
(74) Representative: Simons, Johannes
(86) International application number: PCT/CN2007/000511
(87) International publication number: WO 2008/064536

(57) **Abstract**

An ultrasonic therapeutic device capable of multipoint transmitting includes a substrate (5), on which an ultrasonic transducer array is arranged. Wherein, each of the ultrasonic transducers in the ultrasonic transducer array can independently focus. The device of the present invention has a simple structure and a good focusing performance. There is no interference among the ultrasonic transducers. Therefore, the energy of ultrasonic wave is transmitted by predetermined route with high utilization ratio.

## Description

### FIELD OF THE INVENTION

The present invention pertains to the technical field of medical appliance and relates to an ultrasonic therapeutic device capable of multipoint transmitting.

### BACKGROUND OF THE INVENTION

In recent years, the ultrasonic wave has been used widely in medical imaging detection and non-invasive therapy. By means of experiments, it has been found that the low-energy ultrasonic waves of certain frequency band not only have the thermal effect but also the mechanical effect and bio-chemical effect. Particularly, the experiments and studies in recent years have proved that the bio-chemical effect of ultrasonic waves can not be disregarded, such as its effect on enzyme's activity, accelerating metabolism of cells, stimulating human fibroblast synthesis, etc., especially the low-intensity pulsed ultrasound in combination with cytokine can promote bone marrow mesenchymal stem cells to differentiate into articular cartilage and plays an important role in healing of cartilage injury, therefore, this kind of ultrasound pulse can be applied to treating and accelerating recovery of soft tissue injury, promoting osseous tissue's growth, relieving and healing neuropathic pain. The low-intensity pulsed ultrasound as a means of noninvasive therapy without pain doesn't do any harm to human body; therefore, it has wide application prospects.

The present low-energy ultrasonic therapeutic devices capable of multipoint transmitting, for example, the device disclosed by Japanese Patent No. JP2004-81645A, wherein each single treating unit has no focusing function and ultrasonic waves transmitted from each unit are parallel. Theoretically speaking, "focusing" can be formed in certain area covered by ultrasonic waves, but because of dispersion and interference among plain ultrasonic waves, "focusing" can not be carried out according to pre-settings and the energy depositions with different intensities are formed on acoustic path, owing to which the therapeutic effect can not be achieved, and the health area may be injured. Meanwhile, the energy produced by that device is less and the purpose of promoting tissue's growth and relieving pain cannot be realized. In order to achieve the therapeutic dose during treatment, an external means is needed to carry out multiple covering by ultrasonic waves. The practical operations are difficult for non-professional personnel to grasp them fast and the over-concentrated covering may cause burn.

### SUMMARY OF THE INVENTION

Aiming at the disadvantages in the prior art as mentioned above, the present invention provides a low-energy ultrasonic therapeutic device capable of multipoint transmitting, which has a simple structure and a good focusing performance, and can make it possible to transmit ultrasonic energy along the preset path and has a high use efficiency of ultrasonic energy.

The technical solutions for the problems presented by the present invention are as follows: the ultrasonic therapeutic device capable of multipoint transmitting comprises a substrate, on which an ultrasonic transducer array is arranged. Wherein, each of the ultrasonic transducers in the ultrasonic transducer array can independently focus.

With consideration of simple processing technology, said ultrasonic transducers preferably includes piezoelectric crystal and focusing lens. Said focusing lens is fixed into the pre-set hole on the substrate and piezoelectric crystal is connected to focusing lens by a fixing unit. Wherein, the focusing lens adopts an acoustic lens made of ultrasonic coupling material so as to form an acoustic path for ultrasonic transmission. Said fixing unit may adopt a fastener which makes the piezoelectric crystal be fixed into the pre-set hole on the substrate and the focusing lens is also fixed into the pre-set hole on the substrate by a fastener.

Certainly, the ultrasonic transducer may adopt other forms only if the independent focusing of each ultrasonic transducer can be realized.

The substrate in the present invention can be a flexible substrate. The flexible substrate has good flexibility, which ensures a rapid restoration of pre-set shape of the substrate after a large amount of distortions, and meanwhile it has certain rigidity, which ensures a reliable connection with ultrasonic transducer array without falling off easily. Therefore, the flexible substrate may better adapt to the changes of treatment position and also the changes on the degree of bending through changing the shape of flexible substrate according to severity grade of disease can achieve concentration or dispersing of ultrasonic energy transmitted from multiple ultrasonic transducers.

In the prior art, the ultrasonic waves from each ceramic transducer are transmitted from the whole ceramic surface to the outside and the acoustic field is dispersed. Because the energy transmitted along the direction perpendicular to the transducer surface is the strongest, the ultrasonic waves transmitted may be considered as parallel acoustic waves. Besides the ultrasonic waves in that direction perpendicular to the transducer surface, the ultrasonic waves are dispersed to some extent. When the dispersed ultrasonic waves from multiple ultrasonic transducers are transmitted in the acoustic medium, they will interfere with other ultrasonic waves. That interference will influence the transmission of energy of ultrasonic waves in pre-set linear direction. Each ultrasonic transducer of the ultrasonic therapeutic device of the present invention adopts independent focusing mode. The acoustic fields of all ultrasonic transducers will not interfere with each other and accordingly the energy of the ultrasonic waves can be transmitted along the pre-set path. Therefore, the therapeutic effect can be achieved without other extra operations.

Preferably, an external wrap may be fixed on the substrate oppositely to said transducer array. In the external wrap, there are heat transfer units for removing the heat of each ultrasonic transducer. On said external wrap, there are corresponding holes corresponding to the pre-set holes on the substrate. Each ultrasonic transducer goes through the pre-set holes on the substrate and the corresponding holes on the wrap in turn. Said heat transfer units are flexible bags, which cling to each ultrasonic transducer. There are heat transferring matters contained in the flexible bags. Because the flexible bags have flexibility and toughness, they can cling to the ultrasonic transducers very well and thereby the heat produced by the ultrasonic transducers can be removed fast.

In the present invention, the substrate and the external wrap can be designed in different shapes according to different targets of disease to be treated. The different shapes of the substrate and the external wrap may include strip, sleeve strip, cap, etc. Meanwhile, the pre-set holes for ultrasonic transducer array on the substrate and the external wrap may be round, rectangular, regular polygon, etc. The shape of these holes can be selected specifically according to the structure of ultrasonic transducers.

In order to fix this ultrasonic therapeutic device onto the target of disease conveniently, two bands for fixing flexible substrate may be connected respectively to both ends of the substrate or the external wrap. Two bands may adopt bonding elastic bands, which include one with more burrs and the other with less burrs.

### BRIEF DESCRIPTION OF THE FIGURES

Fig. 1 is a structural diagram of the embodiment 1 of the present invention.
Fig. 2 is a top view of the flexible substrate 5 in Fig. 1.
Fig. 3 is a drawing of partial enlargement of single ultrasonic transducer and fastener 6 in Fig. 1.
Fig. 4 is a structural front view of the upper part of the external wrap 1 in Fig. 1.
Fig. 5 is a top view of Fig. 4.
Fig. 6 is a structural diagram of the embodiment 2 of the present invention.

Wherein: 1-External wrap 2 - Elastic band 3 - Piezoelectric crystal 4 - Acoustic lens 5 - Flexible substrate 6 - Fastener 7 - Flexible bag 8 - Corresponding hole 9 - Pre-set hole 10 - Ultrasonic signal wire 11-Hole for signal wire

### DETAILED DESCRIPTION OF PREFERRED EMBODIMENTS

The present invention will be further explained below in detail with reference to the preferred embodiments and accompanying drawings.

The following embodiments are the non-restrictive embodiments of the present invention.

### Embodiment 1:

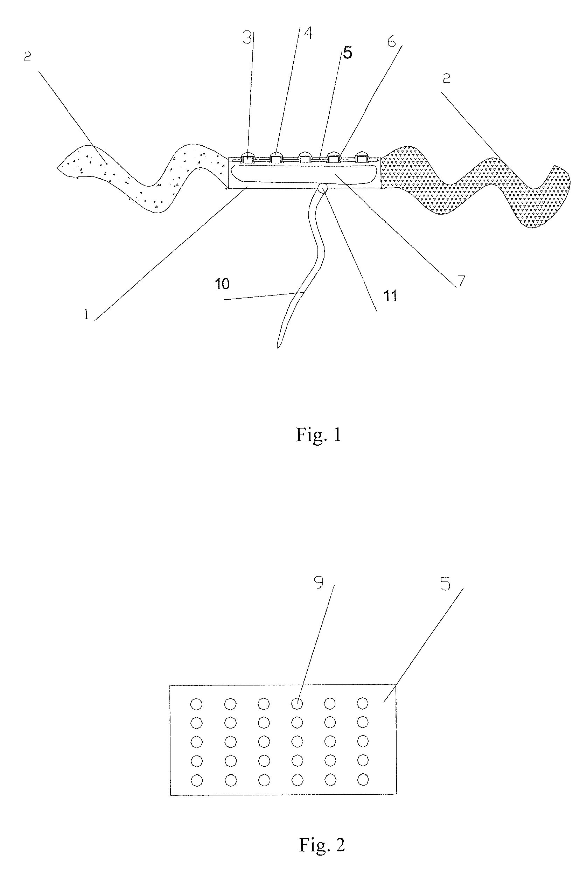

As shown in Fig. 1, the ultrasonic therapeutic device capable of multipoint transmitting of the present embodiment comprises a flexible substrate 5 having pre-set holes 9, an external wrap 1 having corresponding holes 8 corresponding to the pre-set holes 9 on the substrate, an ultrasonic transducer array, an ultrasonic signal wire 10 for providing excitation signals to ultrasonic transducer array and an elastic band 2 for fixing the whole ultrasonic therapeutic device onto the target of disease.

Because the external wrap 1 will contact with skin of patient, the nontoxic external wrap, for example, the wrap made of silicon rubber is adopted. The flexible substrate 5 is fixed on the external wrap 1 by bonding.

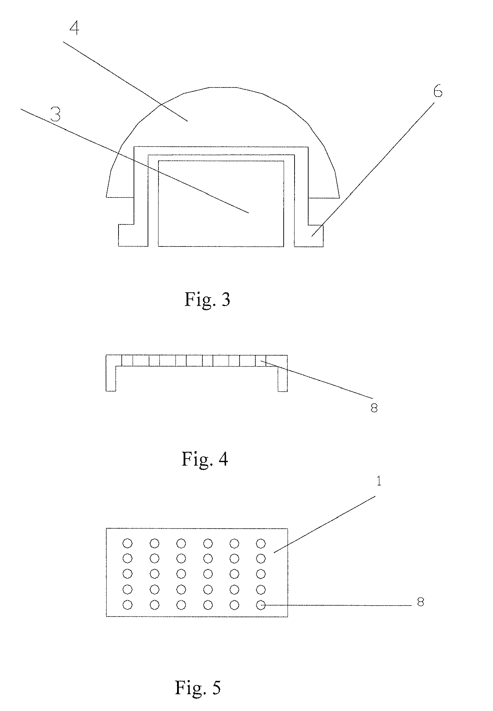

As shown in Fig. 2, in the present embodiment, the shape of the flexible substrate 5 is a rectangle and this shape is suitable for treatment of target of disease on limbs. Accordingly, the shape of external wrap 1 is also a rectangle (as shown in Fig. 4). The pre-set holes 9 on the flexible substrate and the corresponding holes 8 on the external wrap may adopt different shapes. The shape of these holes can be selected specifically according to the structure of each ultrasonic transducer in the ultrasonic transducer array.

The types of ultrasonic transducer can be focusing ultrasonic transducers with all kinds of specifications as needed. As shown in Fig. 3, in the present embodiment, each ultrasonic transducer comprises piezoelectric crystal 3 and acoustic lens 4.

With consideration of simple and convenient processing technology, focusing acoustic lens 4 adopts the acoustic lens made of acoustic coupling material, for example, acoustically transparent rubber. Thus, this acoustic lens can not only focus the ultrasonic waves transmitted from piezoelectric crystal but also form an acoustic transmission path for transmitting the ultrasonic waves. The acoustic lens made of acoustic coupling material is manufactured according to the principle of convex lens on the basis of difference between the acoustic transmission coefficient in the material of which the acoustic lens is made and the acoustic transmission coefficient in acoustic coupling material coated externally and in human body. Therefore, the parallel ultrasonic waves transmitted from piezoelectric crystal 3 can be focused with a set focal length.

For selection of piezoelectric crystals, due to the inverse proportion between the size of piezoelectric crystal and degree of focusing, the stronger the degree of focusing, the smaller the size of piezoelectric crystal. Therefore, for focusing form of the same area, more piezoelectric crystals are needed. For example, for a round ceramic crystal with a radius of 5mm, if its focusing radius is 0.1mm, 25 crystals of the same kind can form a surface focusing region with an area of 0.5X0.5mm².

Piezoelectric crystal 3 is fixed into the pre-set holes 9 on flexible substrate 5 and the corresponding holes 8 on external wrap 1 through a fixing unit, i.e. a fastener 6 and meanwhile, the acoustic lens 4 is also fixed onto flexible substrate 5 by fastener 6. So the fastener 6 can not only fix the ultrasonic transducer, but also fix flexible substrate onto external wrap 1.

Towards flexible substrate 5 and in external wrap 1, there are heat transfer units. In the present embodiment, the heat transfer unit adopts a flexible bag 7, in which there are heat-transferring matters such as heat conduction silicone grease. Because the flexible bag 7 is soft and has toughness, as shown in Fig. 4 and Fig. 5, the flexible bag 7 can cling to piezoelectric crystal 3 in ultrasonic transducer very well through the corresponding holes 8 on the external wrap and the heat produced by the ultrasonic transducers can be removed fast.

In the present embodiment, the bands for fixing flexible substrate 5 adopt bonding elastic bands 2, wherein one has more burrs and the other has less burrs. The bands are connected respectively to both ends of the external wrap 1.

On the external wrap, there is a hole for signal wire 11. The ultrasonic signal wire 10 goes through the hole for signal wire 11 and is connected to each piezoelectric crystal 3 (not illustrated in Fig. 1). All or partial piezoelectric crystals 3 can be excited at the same time to transmit the ultrasonic waves according to the needs.

When using the ultrasonic therapeutic device of the present embodiment, the whole device is fixed on the target of disease of limbs (such as an arm) to treat arthralgia by using the elastic band 2. Then ultrasonic signal wire 10 provides excitation signals to piezoelectric crystal 3 and piezoelectric crystal 3 transmits ultrasonic waves and the ultrasonic waves are focused at a focus by using acoustic lens 4. Because multiple piezoelectric crystals 3 are driven at the same time, multiple focuses are formed at the same time. As long as the crystal is small enough, a linear or slice focusing may be formed at the target of disease. The excitation signals can be adjusted according to the needs and accordingly the ultrasonic intensity can be adjusted, so the ultrasonic therapeutic device of the present invention can promote the tissue's growth rapidly and accordingly relieve the arthralgia.

During the whole process of treatment, the flexible bag 7 keeps absorbing the heat produced by ultrasonic transducers and then radiating the heat. In this way, on the one hand, each ultrasonic transducer is protected from degeneration of performance or damage due to overheating, on the other hand, the patient is protected from skin burning due to overheating of ultrasonic transducer.

The operations of the ultrasonic therapeutic device of the present invention are simple and it is easy and fast for an operator to grasp these operations. Meanwhile, because the ultrasonic transducer of the present invention adopts an array of multiple crystals with a shape of strip or circular disc, the focal points of all ultrasonic transducers will not be concentrated at one point and accordingly the risk of burning the tissue around the target of disease due to excessive energy concentration will not occur.

### Embodiment 2:

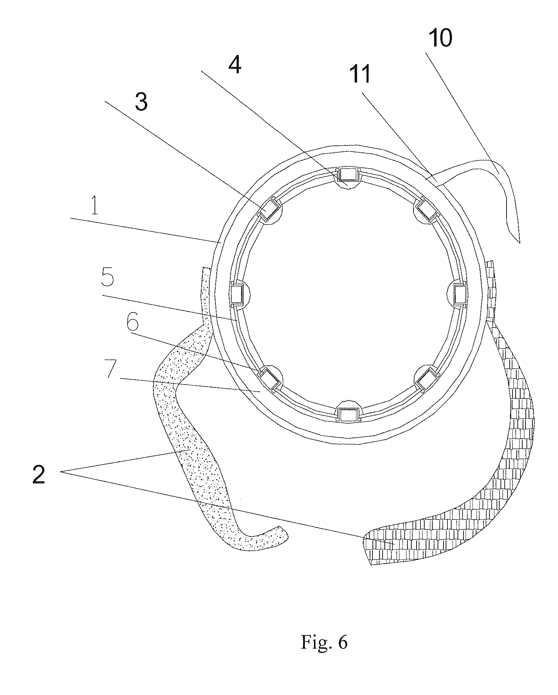

As shown in Fig. 6, the difference between the present embodiment and the embodiment 1 is that the shapes of flexible substrate 5 and the external wrap are round shapes in the present embodiment. The ultrasonic therapeutic device with such structure is suitable to treat the target of disease on the head and may be used to treat neuropathic pain of the head and so on.

Other structures and the methods of use of the present embodiment are the same as those in embodiment 1.

## Claims

1. An ultrasonic therapeutic device capable of multipoint transmitting comprises a substrate, on which an ultrasonic transducer array is arranged; wherein, each of the ultrasonic transducers in the ultrasonic transducer array can independently focus.

2. The ultrasonic therapeutic device capable of multipoint transmitting of claim 1, wherein each ultrasonic transducer comprises piezoelectric crystal and focusing lens; said focusing lens is fixed into the pre-set hole on the substrate and the piezoelectric crystal is connected with focusing lens through a fixing unit.

3. The ultrasonic therapeutic device capable of multipoint transmitting of claim 2, wherein the focusing lens is an acoustic lens which is made of ultrasonic coupling material.

4. The ultrasonic therapeutic device capable of multipoint transmitting of claim 2, wherein said fixing unit may adopt a fastener which makes the piezoelectric crystal be fixed into the pre-set hole on the substrate and the focusing lens is also fixed on the substrate by a fastener.

5. The ultrasonic therapeutic device capable of multipoint transmitting as claimed in any one of claims 1-4, wherein said substrate is a flexible substrate.

6. The ultrasonic therapeutic device capable of multipoint transmitting of claim 5, wherein an external wrap is fixed on the substrate oppositely to said transducer array, and in the external wrap, there are heat transfer units for removing the heat of each ultrasonic transducer.

7. The ultrasonic therapeutic device capable of multipoint transmitting of claim 6, wherein on said external wrap, there are corresponding holes corresponding to the pre-set holes on the substrate, and each ultrasonic transducer goes through the pre-set holes on the substrate and the corresponding holes on the wrap in turn; said heat transfer units are flexible bags which cling to each ultrasonic transducer, and there are heat transferring matters contained in the flexible bags.

8. The ultrasonic therapeutic device capable of multipoint transmitting of claim 7, wherein the heat-transferring matter adopts heat conduction silicone grease.

9. The ultrasonic therapeutic device capable of multipoint transmitting of claim 6, wherein bands for fixing the substrate are connected respectively to both ends of the substrate or external wrap 1.

10. The ultrasonic therapeutic device capable of multipoint transmitting of claim 9, wherein two bands may adopt bonding elastic bands, which include one with more burrs and the other with less burrs.
